(19) Europäisches Patentamt
European Patent Office
Office européen des brevets

(11) **EP 2 255 724 A1**

(12) **EUROPEAN PATENT APPLICATION**

(43) Date of publication:
**01.12.2010 Bulletin 2010/48**

(51) Int Cl.:
***A61B 5/00*** (2006.01)

(21) Application number: **10163395.6**

(22) Date of filing: **20.05.2010**

| (84) Designated Contracting States:<br>**AL AT BE BG CH CY CZ DE DK EE ES FI FR GB GR HR HU IE IS IT LI LT LU LV MC MK MT NL NO PL PT RO SE SI SK SM TR**<br>Designated Extension States:<br>**BA ME RS** | (72) Inventors:<br>• **Asakura, Yoshihiro**<br>  **Hyogo 651-0073 (JP)**<br>• **Kojima, Junko**<br>  **Hyogo 651-0073 (JP)**<br>• **Sato, Toshiyuki**<br>  **Hyogo 651-0073 (JP)** |
|---|---|
| (30) Priority: **25.05.2009 JP 2009124949** | (74) Representative: **HOFFMANN EITLE** |
| (71) Applicant: **SYSMEX CORPORATION**<br>**Kobe-shi,**<br>**Hyogo 651-0073 (JP)** | **Patent- und Rechtsanwälte**<br>**Arabellastrasse 4**<br>**81925 München (DE)** |

(54) **Method for analyzing analyte in tissue fluid, analyzer for analyzing analyte in tissue fluid, cartridge for analyzing analyte in tissue fluid, and kit for analyzing analyte in tissue fluid**

(57)    A method for analyzing an analyte in tissue fluid is disclosed. The method comprises: placing a collecting member in contact with the skin of a subject, wherein the collecting member is configured to collect and retain tissue fluid containing the analyte from the subject; collecting tissue fluid from the subject into the collecting member; detaching the collecting member from the skin of the subject; causing the analyte in the tissue fluid collected by the collecting member to diffuse in a liquid by supplying the collecting member with the liquid; and detecting the analyte diffused in the liquid.

An analyzer for analyzing an analyte in tissue fluid, and cartridge and kit for use in the analyzing method are also disclosed.

FIG. 1

EP 2 255 724 A1

**Description**

FIELD OF THE INVENTION

The present invention relates to a method for collecting tissue fluid containing an analyte from a subject and analyzing the analyte in the collected tissue fluid, an analyzer for analyzing an analyte in tissue fluid, and a cartridge and kit for use in the analyzing method.

BACKGROUND

There are known conventional methods for collecting tissue fluid from the skin of a living body, and measuring the glucose in the collected tissue fluid.

The analyzing method disclosed in WO95/02357 uses a reservoir configured by a two-sided adhesive layer for adhering to the skin of a patient and a cellophane plastic cover adhered to the two-sided adhesive in which a hole is formed, and a measuring unit for measuring the concentration of the glucose collected by a collection medium. The reservoir is adhered to the skin of the patient and water is injected through the hole of the reservoir using a syringe in the analyzing method disclosed in WO95/02357. When a predetermined time elapses, the water injected into the reservoir is periodically collected via the syringe and the collected water is discharged to the measuring unit, which measures the concentration of the glucose contained in the water.

United States Patent Application 2005-0169799 discloses a living body component analyzer provided with a living body component extraction cartridge for extracting a living body component, and an analyzing unit capable of detachably holding the living body component extraction cartridge. The living body component extraction cartridge is provided with an extraction chamber configured by an extraction concavity having an opening on the bottom surface thereof, and a sealing film that covers part of the extraction concavity, and further has a reaction section that communicates with the extraction chamber through a flow path. The reaction section is provided with a sensor member containing an enzyme that is a catalyst for glucose. The analyzing unit is provided with a syringe pump for introducing physiological saline solution into the extraction chamber disposed in the cartridge.

The living body component analyzer performs glucose analysis as follows. The cartridge is first loaded in the analyzing unit, and the living body component analyzer is attached to the skin so that the sealing film on the bottom surface of the cartridge is in contact with the skin of the subject. A space enclosed by the extraction cartridge on the skin of the subject is thus formed. Physiological saline introduced from the syringe pump fills the enclosed space, and glucose is extracted from the skin to the physiological saline. The saline filling the extraction chamber flows through the flow path to the reaction section, and the glucose in the physiological saline reacts with the enzyme in the reaction chamber. The analyzing unit outputs signals based on the reaction product of the glucose and the enzyme, and the amount of glucose is calculated.

The analyzing method disclosed in WO95-02357, however, requires a complex operation of transferring the water from the reservoir to the syringe and then moving the received water to the measuring unit in order to measure the glucose.

On the other hand, the analyzer disclosed in United States Patent Application No. 2005-0169799 is capable of extracting and detecting glucose in a continuous operation without the user performing the complex operation of switching the water. However, the analyzer disclosed in United States Patent Application No. 2005-0169799 does require the unused devices (syringe pump, reaction section) to be continuously fixed to the arm during the extraction period even while glucose is being extracted from the living body.

SUMMARY OF THE INVENTION

The scope of the invention is defined solely by the appended claims, and is not affected to any degree by the statements within this summary.

A first aspect of present invention is: (1) a method for analyzing an analyte in tissue fluid, comprising: placing a collecting member in contact with a skin of a subject, wherein the collecting member is configured to collect and retain tissue fluid containing an analyte from the subject; collecting tissue fluid from the subject into the collecting member; detaching the collecting member from the skin of the subject; causing the analyte in the tissue fluid collected by the collecting member to diffuse in a liquid by supplying the collecting member with the liquid; and detecting the analyte diffused in the liquid.

According to this configuration, a complex operation of moving the collected tissue fluid to a separate container such as a syringe is unnecessary since the tissue fluid is collected from the living body and retained by the collecting member. In this method, only the collecting member is in contact with the skin while tissue fluid is collected, and detection of the analyte contained in the tissue fluid is performed after the collecting member has been removed from the skin.

The devices that are not used during the tissue fluid collection time therefore need not be continuously installed on the skin.

A second aspect of present invention is: (2) an analyzing apparatus for analyzing an analyte in tissue fluid, comprising: an accommodating part configured to accommodate a collecting member containing tissue fluid collected from a subject; a reservoir configured to retain liquid, wherein the reservoir is arranged in communication with the accommodating part; a liquid transporter configured to transport liquid to the accommodating part and to transport liquid to the reservoir from the accommodating part; and a detector for detecting an analyte in the liquid transported into the reservoir by the liquid transporter.

According to this configuration, a complex operation of moving the collected tissue fluid to a separate container such as a syringe is unnecessary since the tissue fluid is collected from the living body and retained by the collecting member. When analyte analysis is performed by this analyzing apparatus, only the collecting member is in contact with the skin while tissue fluid is collected, and detection of the analyte contained in the tissue fluid is performed after the collecting member has been removed from the skin. The devices that are not used during the tissue fluid collection time therefore need not be continuously installed on the skin.

(3) In the analyzing apparatus according to (2), preferably the analyzing apparatus further comprises: a cartridge comprising the accommodating part and the reservoir; and a cartridge holder configured to detachably hold the cartridge.

According to this configuration, the cartridge containing the accommodating part and the reservoir in contact with the liquid containing the analyte can be disposed of after analysis is completed, thus preventing carryover and contamination when the analysis apparatus performs continuous analyses.

(4) In the analyzing apparatus according to (3), preferably, the cartridge comprises a reagent.

According to this configuration, analysis can be readily performed without preparing reagent separate from the cartridge.

(5) In the analyzing apparatus according to (4), preferably the reagent causes a change in optical characteristics by reacting with the analyte in the liquid retained in the reservoir; and the detector is configured to detect the analyte based on the change in the optical characteristics of the reagent.

(6) In the analyzing apparatus according to any one of (2) to (5), preferably the detector comprises an electrode configured to contact the liquid retained in the reservoir.

(7) In the analyzing apparatus according to any one of (2) to (6), preferably the reservoir comprises a first reservoir and a second reservoir; and the detector comprises a first detector for detecting a first analyte in the liquid retained in the first reservoir, and a second detector for detecting a second analyte in the liquid retained in the second reservoir.

According to this configuration, two different types of analyte can be detected in the first reservoir and the second reservoir.

(8) In the analyzing apparatus according to (7), preferably the accommodating part and the first reservoir are connected via a flow path; the second reservoir is disposed in the flow path; and the first reservoir comprises a reagent.

According to this configuration, concern of reagent dissolving in the liquid and influencing the detection of the analyte in the second reservoir is reduced because reagent is provided in the first reservoir on the downstream side among the first reservoir and the second reservoir.

(9) In the analyzing apparatus according to (8), preferably the first detector is disposed opposite a first surface of the cartridge set in the cartridge holder; and the second detector is disposed opposite a second surface of the cartridge set in the cartridge holder.

According to this configuration, the apparatus can be rendered more compact compared to when the first detector and the second detector are provided on the same plane.

(10) In the analyzing apparatus according to any one of (3) to (5), preferably the cartridge has a channel that communicates with the accommodating part and the reservoir when the cartridge is set in the cartridge holder.

According to this configuration, the flow path communicating with the accommodating part and the reservoir is easily formed by disposing the cartridge in contact with the cartridge holder.

(11) In the analyzing apparatus according to any one of (2) to (10), preferably the liquid transporter is configured to move the liquid in the accommodating part to the reservoir by supplying air to the accommodating part.

According to this configuration, the liquid can be moved to the reservoir without changing the concentration of the analyte diffused in the liquid in the accommodating part.

(12) In the analyzing apparatus according to any one of (7) to (9), the analyzing apparatus further comprises a controller for performing analysis relating to the first analyte based on the first analyte detection results obtained by the first detector and the second analyte detection results obtained by the second detector.

A third aspect of present invention is (13) a cartridge configured to be detachably installed in an analyzing apparatus for analyzing an analyte in tissue fluid, the cartridge comprising: an accommodating part configured to accommodate liquid supplied from the apparatus and a collecting member containing tissue fluid collected from a subject so that the collecting member is in contact with the liquid; and a reservoir arranged in communication with the accommodating part, wherein the reservoir is configured to retain liquid transferred from the accommodating part.

According to this configuration, a complex operation of moving the collected tissue fluid to a separate container such as a syringe is unnecessary since the tissue fluid is collected from the living body and retained by the collecting member. In this method, when analyte is analyzed using this cartridge, only the collecting member is in contact with the skin while tissue fluid is collected, and detection of the analyte contained in the tissue fluid is performed after the collecting member has been removed from the skin. The devices that are not used during the tissue fluid collection time therefore need not be continuously installed on the skin.

A fourth aspect of present invention is (14) an analyzing kit for analyzing an analyte in tissue fluid, comprising: a collecting member for collecting and retaining tissue fluid collected from a subject; and the cartridge.

According to this configuration, a complex operation of moving the collected tissue fluid to a separate container such as a syringe is unnecessary since the tissue fluid is collected from the living body and retained by the collecting member. In this method, when analyte is analyzed using this kit, only the collecting member is in contact with the skin while tissue fluid is collected, and detection of the analyte contained in the tissue fluid is performed after the collecting member has been removed from the skin. The devices that are not used during the tissue fluid collection time therefore need not be continuously installed on the skin.

(15) The kit according to (14), preferably the collecting member comprises: a collecting body for collecting and retaining tissue fluid collected from a subject; and an adhesive film for holding the collecting body; wherein the collecting body is accommodated in the accommodating part by adhering the adhesive film to the margin of the opening when the collecting body is inserted through the opening of the accommodating part.

According to this configuration, the collecting member can be maintained in a state of contact with the skin by the adhesive film that adheres the collecting member to the skin. Therefore, the collecting body is fixedly attached to the accommodating part, thus preventing analysis error caused by the collecting body falling from the accommodating part.

BRIEF DESCRIPTION OF THE DRAWINGS

FIG. 1 is an external perspective view of an embodiment of the analyzing apparatus;
FIG. 2 is a schematic view showing the structure of the liquid transporter;
FIG. 3 is a schematic view showing the structure of the liquid discharger;
FIG. 4 is a block diagram of the controller;
FIG. 5 is an external perspective view of the analyzing cartridge;
FIG. 6A is a top view of the collecting member 50;
FIG. 6B is a section view of the collecting member 50;
FIG. 7 is a top view showing the structure of the analyzing cartridge body;
FIG. 8 is a top view showing the structure of the bottom surface of the analyzing cartridge body;
FIG. 9 is a flow chart illustrating the method of analyzing the analyte in the tissue fluid using the analyzing apparatus of the present embodiment;
FIG. 10 is a flow chart illustrating the process of step S7 in FIG. 9;
FIGS. 11A through 11G are schematic views illustrating the operation of the analyzing apparatus of the present embodiment;
FIG. 12 is a schematic view illustrating the blood glucose AUC measurement principle;
FIG. 13 is a schematic view illustrating the blood glucose AUC measurement principle;
FIG. 14 is a top view showing a modification of the analyzing cartridge; and
FIG. 15 is a schematic view showing a modification of the apparatus body.

DETAILED DESCRIPTION OF THE PREFERRED EMBODIMENTS

Hereinafter, an embodiment of a blood analyzer of the present invention will be described in detail with reference to the accompanying drawings.

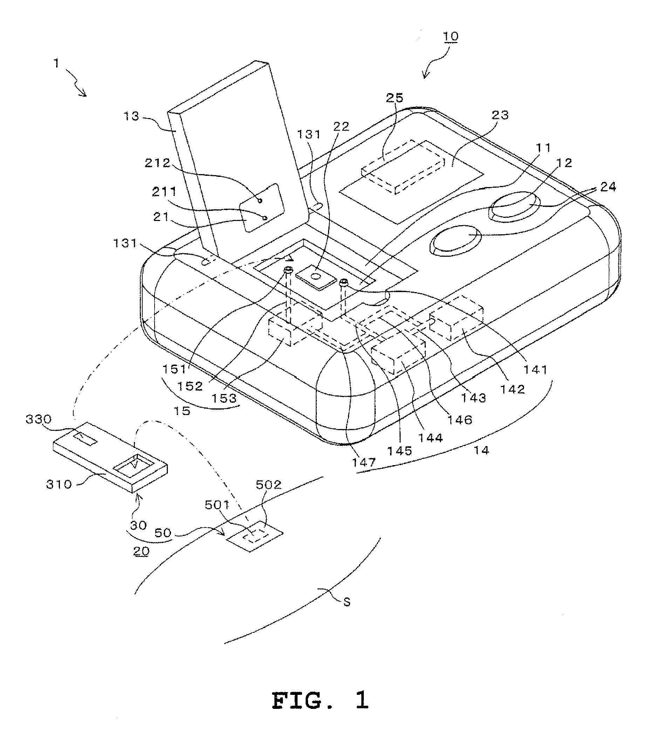

FIG. 1 is an external perspective view of an embodiment of the analyzing apparatus. The analyzing apparatus 1 of the present embodiment is configured by an apparatus body 10 and analyzing kit 20, as shown in FIG. 1. The analyzing kit 20 is configured by an analyzing cartridge 30 and collecting member 50. The analyzing apparatus 1 obtains a value corresponding to the blood glucose concentration AUC (area under the curve) by collecting tissue fluid from a subject via the collecting member, and obtaining the concentration of analytes (glucose and sodium ions) in the tissue fluid in the collecting member. More specifically, the analyzing apparatus 1 is used in the manner described below. Micropores are first formed in the skin of the subject, and the collecting member 50 is adhered to the skin where the micropores have been formed. The collecting member 50 is then removed from the skin of the subject and adhered to the analyzing cartridge 30, whereupon the analyzing cartridge 30 is installed in the cartridge holder 12 of the apparatus body 10, as indicated by the dashed line in FIG. 1. The apparatus body 10 executes predetermined analysis processing of the collecting member adhered to the analyzing cartridge installed in the cartridge holder 12, obtains the glucose concentration in the tissue fluid collected in the collecting member 50, and obtains a value corresponding to the blood glucose AUC.

The structure of the apparatus body 10 is described below with reference to FIG. 1. As shown in FIG. 1, the apparatus body 10 is a thick rectangular housing with a concavity 11 formed in the sheet on the top surface of the housing. The cartridge holder 12 is configured by a concavity that is deeper than the concavity 11, and is disposed within the concavity 11. A cover 13 has the same thickness as the height of the side wall of the concavity 11, and is coupled to the concavity 11. The cover 13 is coupled to the side wall of the concavity 11 by a support shaft 131 extending horizontally from the side wall of the cover 13 and fitted into the side walls of the concavity 11. The cover 13 becomes housed within the concavity 11 when rotated on the support shaft 131 from the state shown in FIG. 1. The cover 13 also becomes unhoused when rotated on the support shaft 131 from the housed state in the concavity 11 to the upright state shown in FIG. 1. The cartridge holder 12 is of sufficient size to be capable of housing the analysis cartridge 30 to be described later.

The cover 13 is supported on the support shaft so as to be forced in the direction of being housed in the concavity 11. The analyzing cartridge 30 disposed in the cartridge holder 12 therefore can be held from above by the cover 13. An injection nipple 141 and discharge nipple 151 provided so as to protrude from the bottom surface of the cartridge holder 12 are flexible members (to be described later) and thus function as cushioning members when pressed by the cover 13. Concern of leakage is reduced since the adhesion of the nipples and induction port and discharge port formed in the analyzing cartridge 30 is made more rigid because the cover 13 presses the analyzing cartridge 30 from above.

The apparatus body 10 has an internal liquid transporter 14 and liquid discharger 15. The liquid transporter 14 is a mechanism for delivering a liquid to the analyzing cartridge 30 loaded in the cartridge holder 12. The liquid discharger 15 is a mechanism for discharging effluent that has been delivered to the analyzing cartridge by the liquid transporter 14.

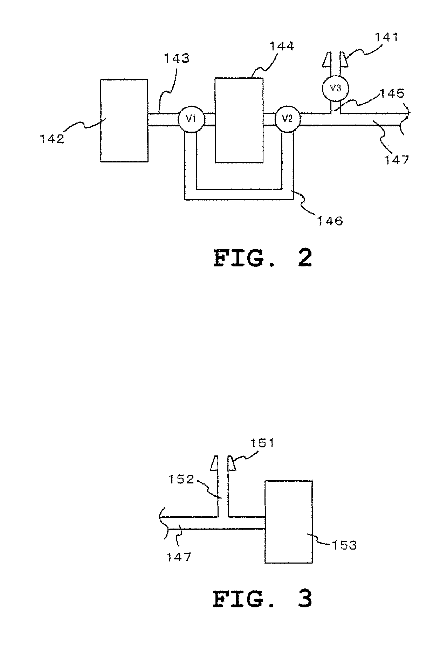

FIG. 2 is a schematic view showing the structure of the liquid transporter. As shown in FIG. 2, the liquid transporter 14 has an injection nipple 141, pump 142, and collection liquid tank 144.

The pump 142 and collection liquid tank 144 are connected by an upstream flow path 143. The collection liquid tank 144 and injection nipple 141 are connected by a downstream flow path 126. The pump 142 and injection nipple 141 are connected by a detour flow path 146 that detours around the collection liquid tank 144. A solenoid valve V1 is provided at the connection point between the upstream flow path 143 and the detour flow path 146. A solenoid valve V2 is provided at the connection point between the downstream flow path 145 and the detour flow path 146. A solenoid valve V3 is provided in the downstream flow path 145 on the downstream side from the connection point of the detour flow path 146. The flow path between the solenoid valves V2 and V3 is a discharge flow path 147 that extends to a discharge tank 153 of the discharger 15 to be described later.

The injection nipple 141 protrudes upward from the cartridge holder 12, and supports the analyzing cartridge 40 from below together with the discharge nipple 151 (to be described later) when the analyzing cartridge 30 is installed in the cartridge holder 12. The analyzing cartridge 30 loaded in the cartridge holder 12 receives injected liquid through the injection nipple 141.

The injection nipple 141 is formed of a flexible member such as rubber or the like. The discharge nipple 151 (described later) is similarly formed. Thus, there is no leakage of liquid because the induction port and discharge port formed in the analyzing cartridge 30 are in gapless contact when the analyzing cartridge 30 is loaded in the cartridge holder 12.

The pump 142 delivers air into the flow path via the operation of a motor that is not shown in the drawing.

The collection liquid tank 144 retains the collection liquid injected into the analyzing cartridge 30. Note that consideration is given to the influence of impurities in the collection liquid on the detection of glucose and sodium ions. Therefore, it is preferable that the collection liquid is substantially free of impurities. From this perspective, pure water is retained as the collection liquid in the liquid collection tank 123 in the present embodiment.

Note that, although not shown in the drawing, the collection liquid tank 144 is configured to be removable from the outside of the apparatus body 10, and replacement of the collection liquid is performed by replacing the collection liquid tank 144.

The solenoid valve V1 operates a valve to switch the connection between the upstream flow path 143 and the collection liquid tank 144, and the connection between the upstream flow path 143 and the detour flow path 146. The solenoid valve V2 operates a valve to switch the connection between the downstream flow path 145 and the collection liquid tank 144, and the connection between the downstream flow path 147 and the detour flow path 146. The solenoid valve V3 operates a valve to switch the operation of the downstream flow path 145.

The liquid transporter 14 delivers a predetermined amount of the collection liquid retained in the collection liquid tank 144 to the analyzing cartridge 30 by pumping air from the pump 141 via the configuration described above. The specific operation of the liquid transporter 14 will be described later.

FIG. 3 is a schematic view showing the structure of the liquid discharger 15. As shown in FIG. 3, the discharger 15 has a discharge nipple 151, flow path 152, and liquid discharge tank 153. The discharge nipple 151 protrudes upward from the cartridge holder 12, and supports the analyzing cartridge 30 from below together with the previously mentioned injection nipple 141 when the analyzing cartridge 30 is installed in the cartridge holder 12. The collection liquid delivered to the analyzing cartridge 30 by the liquid transporter 14 is taken through the discharge nipple 151.

The liquid discharge tank 153 is connected to the discharge nipple 151 through the flow path 152, and retains the collection liquid (effluent) taken from the discharge nipple 151. Note that, although not shown in the drawing, the liquid discharge tank 153 is configured to be connectable outside the apparatus body 10 so as to discharge the effluent outside the apparatus body 10.

The apparatus body 10 is further configured by a glucose detector 21, sodium detector 22, display part 23, operation part 24, and controller 25.

The glucose detector 21 is disposed on the back surface of the cover 13. That is, the glucose detector 21 is provided on the surface of the cover 13 that faces the cartridge holder 12 when the cover 13 is housed in the concavity 11. The glucose detector 21 has a light source 211 for emitting illumination light, and a light receiver 212 for receiving the reflected light emitted from the light source 211. Thus, the glucose detector 21 is configured to irradiate light on the analyzing cartridge 30 installed in the cartridge holder 12, and receive the reflected light from the irradiated analyzing cartridge 30. The analyzing cartridge 30 includes a glucose reactor 330 that changes color through chemical reaction with the glucose in the tissue fluid collected from a living body in a manner to be described later. The glucose detector 21 detects the change in the light absorbance of the glucose based on the reflected light, and quantifies the glucose from the obtained reflected light.

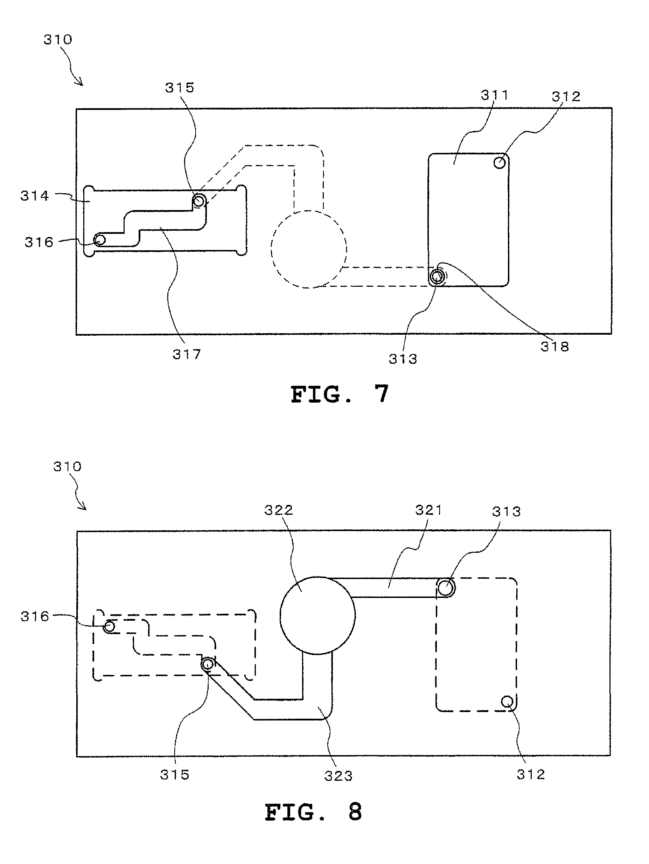

The sodium detector 22 is disposed on the bottom surface of the cartridge holder 12. The sodium detector 22 has a rectangular plate-like member on the bottom surface of the cartridge holder 12, and a pair of electrodes for measuring sodium ion concentration disposed in the approximate center of the plate-like member. The sodium ion concentration measuring electrodes include sodium ion selective electrode formed of silver/silver chloride and provided with a sodium ion selective film, and an opposed electrode formed of silver/silver chloride. The surface of the plate-like member is covered by a flexible material that functions as a cushion member when the analyzing cartridge 30 installed in the cartridge holder 12 is pressed by the cover 13. The plate-like member forms a space by enclosing the concavity on the bottom surface of the analyzing cartridge 30 (first connecting flow path, sodium detection retainer, second connecting flow path (refer to FIGS. 7 and 8)), and this space functions as a flow path for the passage of the liquid. From the perspective of preventing liquid leakage, it is therefore desirable that the analyzing cartridge 30 and plate-like member make gapless contact. In the present embodiment, the adhesion between the analyzing cartridge 30 and the plate-like member is improved by coating the surface of the plate-like member the flexible material, and pressing down on the analyzing cartridge 30 from above via the cover 13.

The display part 23 is provided on the top surface of the apparatus body 10, and includes a liquid crystal panel. The display part 23 functions to display an operation screen for the user performing operations, and display measurement results when measurements have been completed.

The operation part 24 is disposed on the top surface of the housing of the apparatus body 10, and includes a plurality of buttons. A user specifies the start of a measurement, shutdown and the like to the controller 25 by performing operations via these buttons.

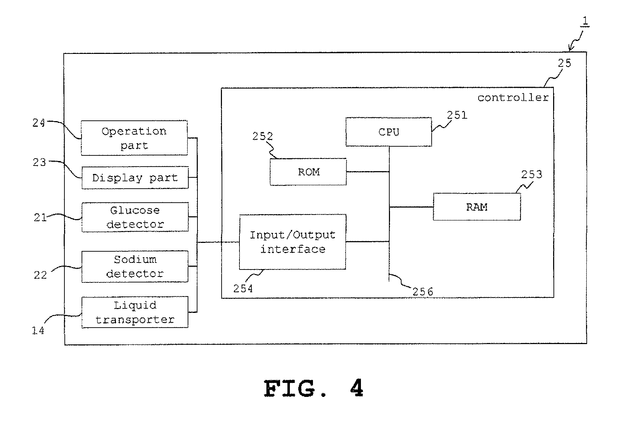

The controller 25 is provided within the apparatus body 10. Fig 4 is a block diagram indicating configuration of controller 25 of the present embodiment. Controller 25 comprises CPU 251, ROM 252, RAM 253 and input/output interface 254. These components are respectively connected to each other via bass 256. Controller 25 is connected to each of the mechanisms (operation part 24, display part 23, glucose detector 21, sodium detector 22 and liquid transporter 14). The CPU controls the operations of the mechanisms by reading and executing programs stored in the ROM 252. The RAM 253 is used as a program development area when executing the programs stored in the ROM 252.

The structure of the analyzing kit 20 is described below. As shown in FIG. 1, the analyzing kit 20 of the present embodiment is configured by the analyzing cartridge 30, and collecting member 50 adhered to the analyzing cartridge 30. The analyzing kit 50 provides the collecting member 50 and analyzing cartridge 30 as separate bodies before use in analysis, and the collecting member 50 is adhered to the analyzing cartridge 30 when to be used for analysis.

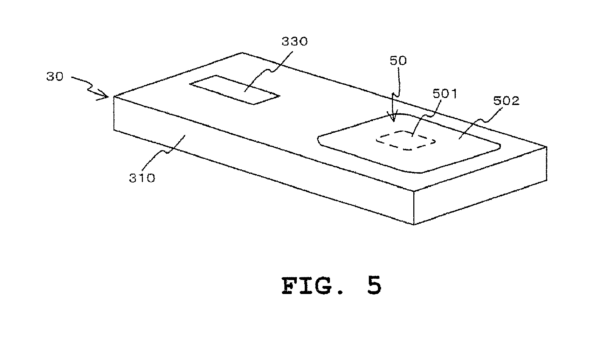

FIG. 5, is a perspective view showing the collecting member 50 adhered to the analyzing cartridge 30. As shown in FIG. 5, the analyzing cartridge 30 mainly has a cartridge body 310 and glucose reactor 330. Note that the structural details of the cartridge body 310 are omitted in FIG. 5.

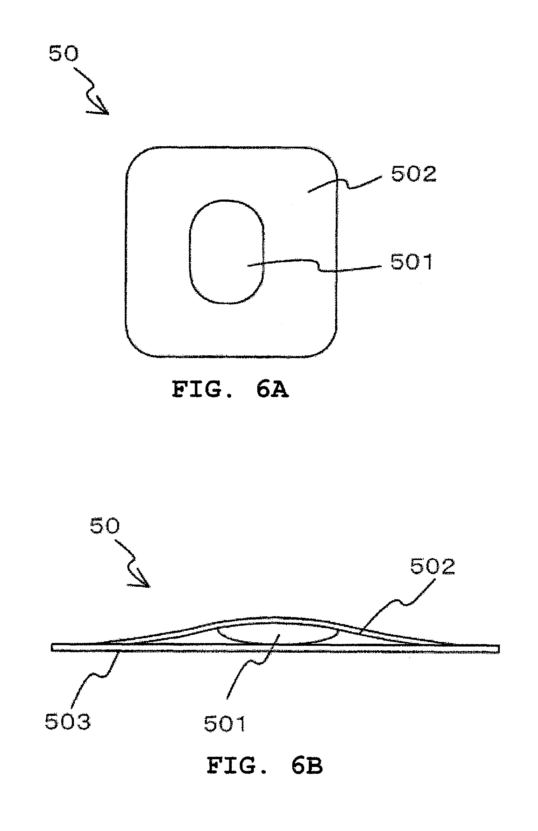

The collecting member 50 is described below with reference to FIG. 6, and the analyzing cartridge 30 is described below with reference to FIGS. 5, 7, and 8.

FIG. 6A is a top view showing the structure of the collecting member 50. In FIG. 6A, the bottom surface of the collecting member 50 (the surface in contact with the skin in FIG. 1) is exposed. In the following description, the surface of the collecting member 50 contacting the skin is the bottom surface and the back panel is the top surface.

The collecting member 50 has a gel 501 and adhesive film 502, and is configured so that the gel 501 is maintained in the approximate center of the adhesive film 502.

While the gel 501 is maintained in this state, the gel has water retention characteristics and is capable of retaining the tissue fluid collected from the skin of the subject. The gel 501 collects the tissue fluid that contains analytes (glucose and sodium ions) from the living body. Thus, the gel 501 does not substantially contain either glucose or sodium ions among its constituents. In the present embodiment, the gel 501 is polyvinyl alcohol. The gel 501 of the present embodiment is formed in an approximate rectangular shape measuring 12 mm (length) x 7 mm (width) x 0.7 mm (thickness).

The adhesive film 502 is a member that does not transmit moisture and has a bottom surface with adhesive and a top surface without adhesive. The adhesive film 502 is an approximate square shape measuring 28 x 28 mm. The bottom surface with the adhesive is adhered to the skin of the subject and the cartridge body 310. The gel 501 is maintained in the approximate center of the adhesive film 502 by the adhesion to the bottom surface of the adhesive film 502.

The adhesive film 502 can be adhered to the skin while maintaining the gel 501 via the surface with the adhesive characteristics. Evaporation of the moisture collected by the gel 501 can be prevented by the material of the adhesive film that does not transmit moisture. Hence, the gel 501 prevents a change in the surface area in contact with the skin and evaporation while the gel 501 collects the tissue fluid, thus preventing fluctuation in the tissue fluid collection efficiency. Via its size, the adhesive film 502 seals the opening so as to prevent leakage of liquid from the opening in the gel accommodating part 311 (refer to FIG. 7) when the cartridge body 310 is positioned on the gel accommodating part 311.

FIG. 6B is a sectional view showing the collecting member 50 prior to use. The adhesive film 502 is adhered to a peelable sheet 503 in the pre-use state, and the adhesive surface and gel 501 are exposed by peeling the peelable sheet 503 for use.

The analyzing cartridge 30 is described below. As shown in FIG. 5, the analyzing cartridge 30 is mainly configured by a glucose reactor body 330, and cartridge body 310.

The glucose reactor body 330 contains reagents, including an enzyme (glucose oxydase (GOD)) catalyst for the glucose, an enzyme (peroxidase (POD)) catalyst for hydrogen peroxide ($H_2O_2$) generated from the glucose via the presence of GOD, and color-producing agent tetramethylbenzidine (3,3',5,5'-tetramethylbenzidlne)For reacting with the activated oxygen (O*) generated from the $H_2O_2$ via the presence of POD. The glucose reactor body 330 is of sufficient shape and size as to be capable of fitting in the reagent holder 314 provided in the cartridge body 310 to be described later.

The cartridge body 310 is configured by a rectangular acrylic plate measuring 24 mm (width) x 56 mm (length) x 3 mm (thickness), the surface of which is provided with a concavity (reagent holder 314, refer to FIG. 7) into which the glucose reactor body fits. The gel 501 is adhered to the surface of the collecting member 50 disposed in the concavity (gel accommodating part 311, refer to FIG. 7) formed in the surface of the cartridge body 310 as will be described later. In the following description, the surface of the cartridge body 310 on which the collecting member 50 is adhered (exposed surface in FIG. 5) is the top surface, and the opposite surface is the bottom surface. The structure of the cartridge body 310 is described in detail below with reference to FIGS. 7 and 8.

FIGS. 7 and 8 are top plan views of the cartridge body 310. In FIG. 7, the structure of the top surface of the cartridge body 310 is indicated by a solid line, whereas the structure of the bottom surface is indicated by a dashed line. In FIG. 8, the structure of the bottom surface of the cartridge body 310 is indicated by a solid line, whereas the structure of the top surface is indicated by a dashed line.

As shown in FIGS. 7 and 8, the cartridge body 310 is structurally configured by a gel accommodating part 311, induction port 312, upstream connection port 313, reagent holder 314, glucose detection reservoir 317, downstream connection port 315, and discharge port 316, as viewed from the top. The cartridge body 310 also structurally has a first connection flow path 321, sodium detection reservoir 322, and second connection path 323, as viewed from the bottom. These structures can be entirely obtained by depressions or pass-throughs. Hence, the cartridge body 310 of the present embodiment can be manufactured as an integrated single unit using well known and simple methods such as injection molding.

According to this structure, the cartridge body 310 forms a single flow path from the induction port 312 to the discharge port 316 when the cartridge body 310 is installed in the cartridge holder 12 of the apparatus body 10.

The structure of the cartridge body 310 along the fluid flow is described below.

The induction port 312 is a 0.7 mm diameter hole through the cartridge body from the top surface to the bottom surface. The induction port 312 is disposed so that the injection nipple of the cartridge holder 12 is directly therebelow in a vertical direction when the cartridge body 310 is installed in the cartridge holder 12 of the apparatus body 10. Thus, the collection liquid is injected from the injection nipple 141 and through the induction port 312 into the gel accommodating part 311 when the cartridge body 310 is loaded in the cartridge holder 12.

The gel accommodating part 311 is a rectangular concavity measuring 14 mm (large side) $\times$ 9 mm (narrow side) formed on the top surface of the cartridge body 310. The previously mentioned induction port 312 is provided on the bottom surface of the gel accommodating part 311.

The gel accommodating part 311 accommodates the gel 501 measuring 12 mm (large side) × 7 mm (narrow side) × 0.7 mm (thickness) maintained on the previously mentioned collecting member 50 via the above described size.

The gel accommodating part 311 accommodates the gel 501 maintained on the collecting member 50. Specifically, the adhesive film 502 of the collecting member 50 is adhered to the margin of the gel accommodating part 311, and the maintained gel 501 is suspended on the gel accommodating part 311 when the collecting member 50 is disposed in the gel accommodating part 311.

A step 318 configured by a depression deeper than the bottom surface of the gel accommodating part 311, and the upstream connection port 313 are disposed at positions on a diagonal line of the induction port 312 on the bottom surface of the gel accommodating part 311. The upstream connection port 313 is a 1.5 mm diameter hole passing through the cartridge body 310 from the top surface to the bottom surface.

As shown in FIG. 8, the upstream connection port 313 is an opening on the bottom surface of the first connection flow path 321 configured by a channel having a depth of approximately 0.5 mm formed on the bottom surface. The first connection flow path 321 extends horizontally in the longitudinal direction of the cartridge body 310, and connects to the sodium detection reservoir 322 formed in the approximate center of the bottom surface of the cartridge body 310.

The sodium detection reservoir 322 is a circular channel having an approximate depth of 1.5 mm. The sodium detection reservoir 322 is provided to be positioned in a vertical direction directly above the sodium detector 22 provided on the bottom surface of the cartridge holder 12 when the analyzing cartridge 30 is loaded in the cartridge holder 12 of the apparatus body 10. More specifically, the sodium detection reservoir 322 is disposed such that the sodium selective electrode of the sodium detector 22 is positioned in the space circumscribed by the plate-like member and the sodium detection reservoir 322.

The sodium detection reservoir 322 connects to the second connection path 323 which is configured by a channel having an approximate depth of 0.5 mm. The second connection path 323 extends horizontally from the starting point of the sodium detection reservoir 322 through the step in the latitudinal direction of the cartridge body 310, then changes direction 90 degrees and extends horizontally in the longitudinal direction, and subsequently changes direction 45 degrees and extends horizontally to the interior side.

The downstream connection port 315 is formed on the base of the terminal end of the second connection path 323. The downstream connection port 315 is a 1.7 mm diameter hole passing through the cartridge body 310 from the top surface to the bottom surface.

Returning to FIG. 7, a reagent holder 314 is provided on the top surface of the cartridge body 310. The reagent holder 314 is configured by a concavity having an approximate depth of 1 mm formed on the top surface of the cartridge body 310. The reagent holder 314 has an approximate rectangular shape extending on the longitudinal direction of the cartridge body 310.

The reagent holder 314 is provided with a discharge path 317 configured by a channel deeper than the reagent holder 314. The downstream connection port 315 is open to the bottom surface of the discharge path 317. The discharge path 317 is configured by a channel having an approximate depth of 1.5 mm from the bottom surface of the reagent holder 314. The discharge path 317 extends horizontally from the starting point of the position of the open downstream connection port 315 latitudinal direction of the cartridge body 310, then changes direction 90 degrees and extends horizontally in the longitudinal direction, and subsequently changes direction 90 degrees and extends horizontally to the latitudinal direction, whereupon the direction again changes 90 degrees and extends horizontally in the longitudinal direction. A discharge port 316 is formed at the terminal end of the discharge path 317.

The discharge port 316 is a 0.7 mm diameter circular hole passing through the cartridge body 310 from the top surface to the bottom surface. The discharge port 316 is disposed so that the discharge nipple 151 of the cartridge holder 12 is positioned directly therebelow in a vertical direction when the cartridge body 310 is installed in the cartridge holder 12 of the apparatus body 10. Thus, the discharge nipple 151 discharges collection liquid through the discharge port 316 when the cartridge body 310 is installed in the cartridge holder 12.

As can be clearly understood from the above description, the induction port 312 and the upstream connection port 313 are connected within the gel accommodating part 311. The upstream connection port 313 and downstream connection port 315 are connected through the first connection path 321, sodium detection reservoir 322, and second connection path 323. More specifically, the upstream connection port 313 and downstream connection port 315 are connected by a series of channels having a width and depth that allows the passage of the liquid between the horizontal surface and the bottom surface of the cartridge body 310 when the cartridge body 310 is disposed on the horizontal surface. The downstream connection port 315 and the discharge port 316 are connected through the discharge path 317.

Hence, the cartridge body 310 configures a single flow path from the induction port 312 to the discharge port 316 when placed on the horizontal surface.

The function of the analyzing cartridge 30 of the present embodiment is described below in conjunction with the operation of the apparatus body 10.

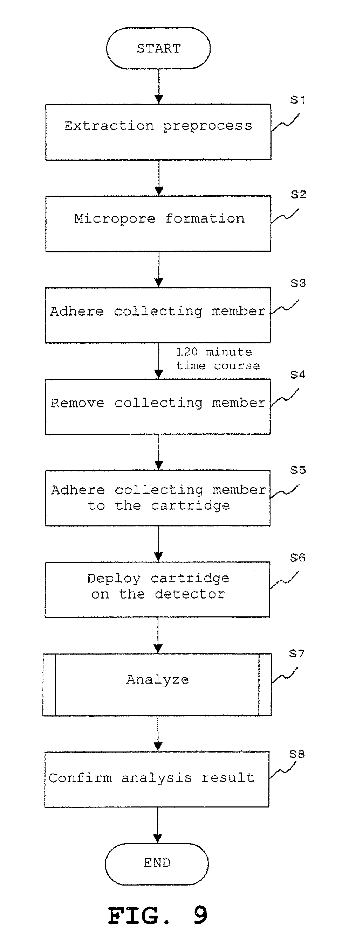

FIG. 9 is a flow chart illustrating the method of analyzing analyte in tissue fluid using the analyzing apparatus of the present embodiment.

In step S1, the subject performs preprocessing operation on the region from which tissue fluid is to be collected, that is, the subject washes the region with alcohol. Material (sweat, dirt and the like) adhered to the skin that might interfere with analysis is thus removed.

In step S2, micropores are formed in the collection region that was washed with alcohol. Micropores are holes piercing through the stratum corneum of the skin and into the dermis to the boundary of the corium, but not extending so far as the deep dermis. The process of forming micropores can be performed using the micropore forming apparatus disclosed in US Patent Application Publication No. 2007-0233011. Tissue fluid is this collected from the subdermal tissue without extracting blood.

In step S3, the collecting member 50 is adhered to the collection region in which the micropores are formed. More specifically, the gel 501 in the collecting member 50 is disposed relative to the skin so as to cover the region in which micropores are formed, and the adhesive film 502 holding the gel 501 is adhered to the margin of the region in which micropores are formed. The collecting member 50 is maintained thusly adhered to the skin for a predetermined time, for example 120 minutes or more, and tissue fluid flows from the skin through the micropores and is collected in the gel 501.

After the elapse of the predetermined time during which the collecting member 50 has been adhered to the skin, the process advances to step S4 and the collecting member 50 is removed from the skin.

In step S5, the collecting member that has been removed from the skin is adhered to the analyzing cartridge 30. Details of adhering the collecting member 50 to the analyzing cartridge 30 have been described previously.

In step S6, the analyzing cartridge 30 to which the collecting member 50 has been adhered is installed in the cartridge holder 12 of the apparatus body 10. At this time, the analyzing cartridge 30 is disposed so that the sodium detection reservoir 322 is opposite the bottom surface of the cartridge holder 12.

After the analyzing cartridge 30 is installed in the apparatus body 10, the cover 13 is closed on the apparatus body 10. The analyzing cartridge 30 installed in the cartridge holder 12 is interposed between the injection nipple 141, discharge nipple 151, and sodium detector 12 and cover 13 and pressed from above by the cover 13 which exerts a force in the direction of closure.

In step S7, the analytes in the collected tissue fluid are analyzed. Specifically, with the analyzing cartridge 30 installed in the cartridge holder 12, the user operates the operation unit 24 of the apparatus body 10 to instruct the controller 25 to start the analysis. The controller 25 receives the instruction and performs analysis of the living body components by executing a predetermined program. The analysis includes obtaining the glucose concentration via cooperation between the controller 25 and glucose detector 21, and obtaining the sodium ion concentration via cooperation between the controller 25 and the sodium detector 22. This process is described later.

When the analysis is completed by the apparatus body 10, the analysis results are displayed on the display unit 23. The subject confirms the displayed analysis results in step S8, and the series of operation ends.

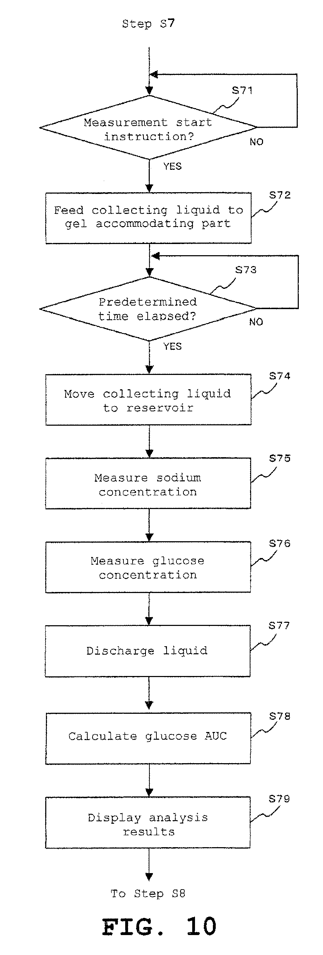

FIG. 10 is a flow chart illustrating the process of step S7 in FIG. 9. Programs for executing the processes described above are stored in the ROM of the controller 25, and the CPU of the controller 25 executes the processes shown in the flow chart by executing the programs stored in the ROM.

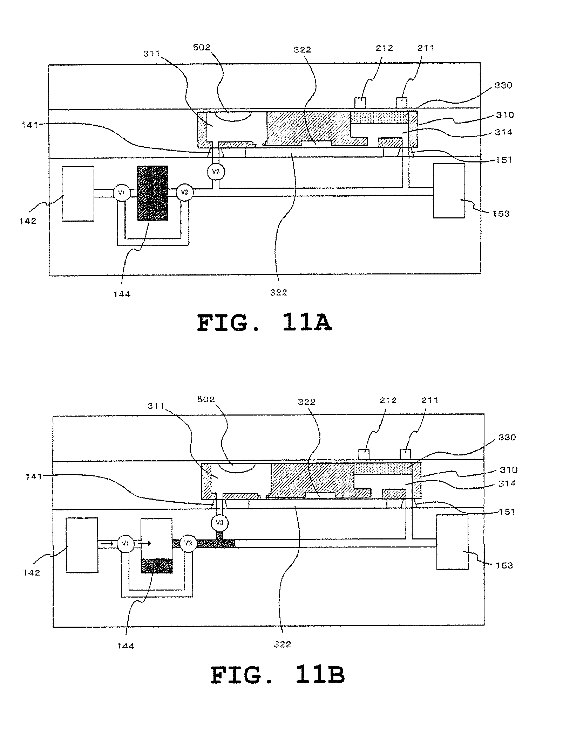

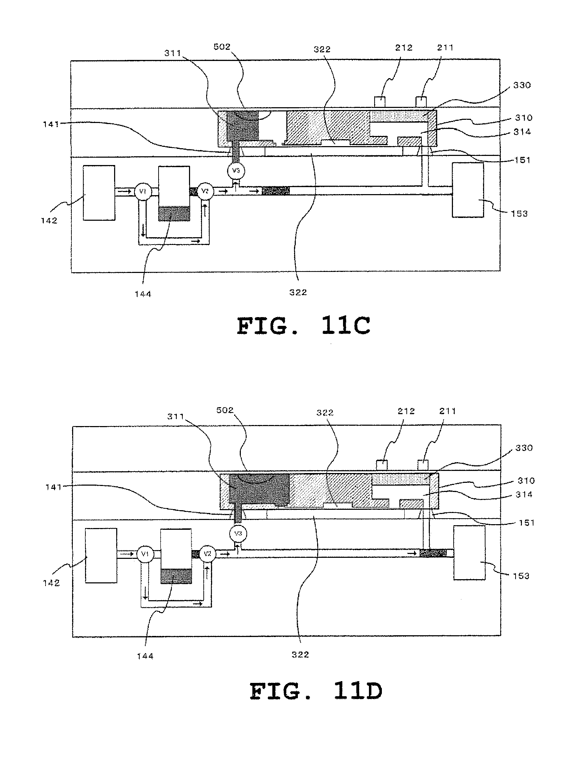

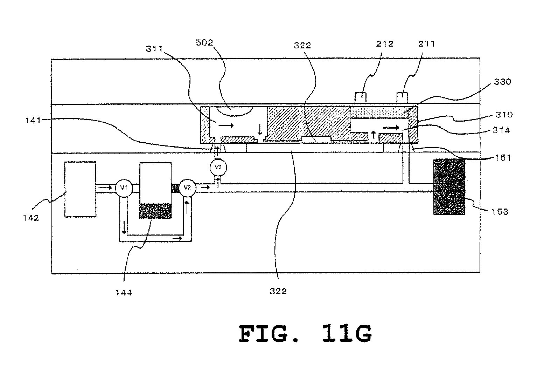

FIGS. 11A through 11G are schematic cross sectional views illustrating the operation of the analyzing apparatus of the present embodiment, and the time course of the flow of the collection fluid is indicated in the sequence A through G. Note that in FIGS. 11A through 11G the analyzing cartridge 30 is shown installed in the cartridge holder 12, and the flow of air delivered by the pump 142 is indicated by the arrow while the flow of the collection fluid is indicated by the diagonal lines. The following description pertains to the operation of the analyzing apparatus 1 with reference to FIGS. 11A through 11G.

In step S71, the controller 25 determines whether a user-issued analysis start instruction has been received. When the controller 25 determines that an analysis start instruction has been received (step S71: YES), the process continues to step S72. When the controller 25 determines that an analysis start instruction has not been received (step S71: NO), the process returns to step S71.

In step S72, the controller 25 executes a process for injecting collection liquid to collect the analyte in the tissue fluid collected in the gel 501. Specifically, the controller 25 executes processes to connect the upstream path 143 and the pump 142 by controlling the solenoid valve V1, connect the pump 142 and the downstream path 145 by controlling the solenoid valve V2, and closes the connection between the downstream path 145 and the injection nipple 141 by controlling the solenoid valve V3. The controller 25 then executes a process to pump air to the flow path by actuating the pump 142 via a motor not shown in the drawings. Thus, the collection liquid reserved in the collection liquid tank 144 is pushed to the downstream path 145 and the discharge path 147, and the collection liquid fills the flow path from the solenoid valve V2 to the solenoid valve V3 via the pushed collection liquid.

The controller 25 then stops the actuation of the pump 142, and subsequently executes processes to connect the upstream path 143 and detour path 146 by controlling the solenoid valve V1, connecting the detour path 146 and the downstream path 145 by controlling the solenoid valve V2, and opening the connection between the downstream path 145 and the injection nipple 141 by controlling the solenoid valve V3. Thus, a continuous flow path is formed from the pump 142 to the injection nipple 141 through the detour path 146. The controller 25 then executes a process to reactuate the pump 142 and pump air into the flow path. Thus, the air pumped by the pump 142 is driven toward the injection nipple 141 through the upstream path 143, detour path 146, and downstream path 145. At the point in time of switching the open/closed state of the solenoid valves V2 and V3, the flow path from the solenoid valve V2 to the solenoid valve V3 is filled with a predetermined amount of collection liquid. The predetermined amount of collection liquid retained between the solenoid valves V2 and V3 is pushed toward the injection nipple 141 by the air pumped by the pump 142, and the collection liquid in the discharge path 147 is pushed toward the discharge liquid tank 153 (refer to FIG. 11C).

The injection nipple 141 is disposed so as to communicate with the induction port 312 of the analyzing cartridge 30 installed in the cartridge holder 12, and the collection liquid moving toward the injection nipple 141 is injected into the gel accommodating part 311 through the injection nipple 141 and the induction port 312.

When injecting the collection liquid into the gel accommodating part 311, the gel accommodating part 311 is filled with the injected collection liquid. Note that although the collection liquid injected into the gel accommodating part 311 moves the shortest distance from the induction port 312 toward the upstream connection port 313, the collection liquid is maintained, via surface tension, at the step 318 before leaking from the upstream connection port 313 because the margin of the upstream connection port 313 is circumscribed by the step 318 which is one level higher than the opening of the upstream connection port 313. Hence, the injected collection liquid spreads to the entirety of the gel accommodating part 311 without leakage from the upstream connection port 313because the collection liquid is subjected to pressure in a direction to disperse to the entirety of the gel accommodating part and away from the direction of leaking from the upstream connection port 313 insofar as the pressure is not increased above the level of the surface tension by the effect of the step 318. (Refer to FIG. 11C). Thus, it is possible to prevent a decrease in analysis precision caused by insufficient filling of the collection liquid in the gel accommodating part 311.

When the predetermined amount of collection liquid is injected into the gel accommodating part 311, the gel 501 is embedded in the collection liquid (refer to FIG. 11C). The controller 25 temporarily stops the actuation of the pump 142 at the moment of completing the injection of the predetermined amount of collection liquid. Even though the actuation of the pump 142 is stopped, the collection liquid does not reverse flow from the induction port 312 due to the effect of the air pressure from below on the liquid at the surface of the induction port 312. Since the flow path maintains constant air pressure by stopping the actuation of the pump 142, the collection liquid that has filled the gel accommodating part 311 does not leak from the upstream connection port 313 and is maintained within the gel accommodating part 311.

In step S73, the controller 25 determines whether a predetermined time (for example, 10 minutes) has elapsed since the end of the injection of the collection liquid. When the controller 25 determines that the predetermined time has elapsed (step S73: YES), the process continues to step S74, whereas the process returns when the controller 25 determines that the predetermined time has not elapsed (step S73: NO), and the process of step S74 is repeated until the predetermined time has elapsed.

The tissue fluid collected in the gel 501 moves (disperses) into the collection liquid when the gel 501 is maintained in the embedded state in the collection liquid for the predetermined time.

In step S74, the controller 25 executes a process to move the collection liquid retained in the gel accommodating part 311 to the sodium detection reservoir 322 and the glucose detection reservoir 314. Specifically, the controller 25 controls the pump 124 so as to pump air of the same volume as the gel accommodating part 311 into the gel accommodating part 311. When air is pumped into the gel accommodating part 311, the pressure increases above the surface tension of the liquid on the surface of the upstream connection port 313, and the collection liquid retained in the gel accommodating part 311 flows through the upstream connection port 313 to the first connection flow path 321.

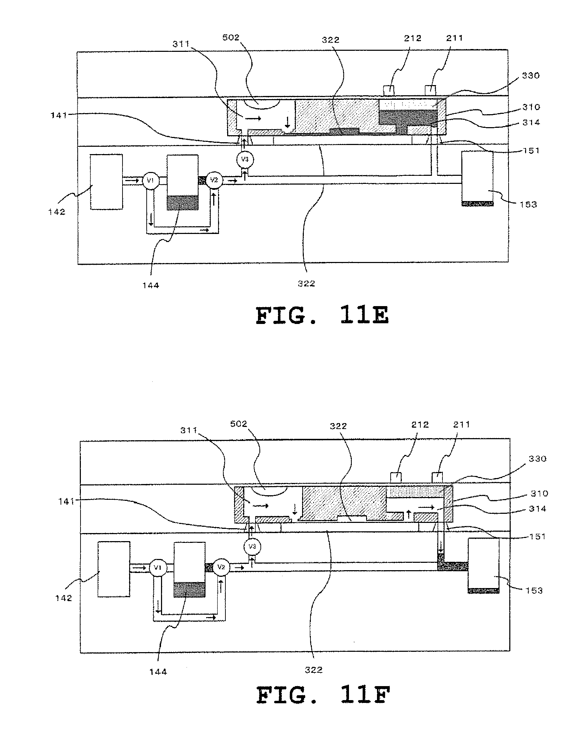

When air is pumped to the gel accommodating part 311, the collection liquid that flowed to the first connection path 321 arrives at the sodium detection reservoir 322 and fills the sodium detection reservoir 322. When air is pumped into the gel accommodating part 311, the collection liquid also arrives at the glucose detection reservoir 317 through the downstream connection port 315. The collection liquid pumped to the glucose detection reservoir 317 comes into contact with the glucose reactor 330 maintained in the reagent holder 314 (refer to FIG. 11E).

The collection liquid is thus mixed by the configuration in which the collection liquid retained in the gel accommodating part 311 is delivered to a different position in the gel accommodating part 311. Hence, the concentration of the analyte in the collection liquid becomes uniform via the mixing of the collection liquid even though the analyte moved to the gel accommodating part 311 was unevenly diffused in the collection liquid.

In step S75, the controller 25 executes processes for stopping the actuation of the pump 141, and measuring the sodium concentration.

As shown in FIG. 11E, the sodium detector 22 provided on the bottom surface of the cartridge holder 12 is configured by an enclosed space with the sodium detection reservoir provided on the bottom surface of the analyzing cartridge 30. The sodium detection reservoir 322 has a sodium ion concentration measuring electrode disposed so as to be exposed on the surface. The sodium ion concentration measuring electrode is completely immersed in the retained collection liquid when the collection liquid is retained in the sodium detection reservoir 322. In this state, the controller 25 applies a constant current to the sodium ion concentration measuring electrode and obtains a voltage value, then obtains the sodium ion concentration CNa based on the obtained voltage value and a calibration curve prestored in the controller 25.

In step S76, the controller 25 executes a process for measuring the glucose concentration.

The light source 121 and light receiver 122 are provided on the surface opposite the cover 13 of the analyzing cartridge 30. As shown in FIG. 11E, the glucose reactor 330 is held by the reagent holder 314 of the cartridge body 310, and the glucose reactor 330 is immersed in the collecting liquid. The glucose that moved to the collection liquid produces chemical reactions described below via the GOD, H2O2, POD, and color-producing agent contained in the glucose reactor, and as a result the glucose reactor 330 changes color.

$$\text{Glucose} + O2 + H2O \rightarrow \text{(catalyst via GOD)} \rightarrow \text{gluconic acid} + H2O2$$

$$H2O2 + \text{Color-producing agent} \rightarrow \text{(catalyst via POD)} \rightarrow 2H2O + \text{color-producing agent (oxidation + color change)}$$

As can be understood from the chemical reaction equations above, the degree of coloration produced by the color-producing agent is proportional to the amount of glucose. Thus, the glucose concentration can be obtained by optically detecting the degree of color change of the color-producing agent.

The present embodiment is configured so that light of a wavelength efficiently absorbed by the color following the color change of the color-producing agent is emitted from the light source 121 and irradiates the glucose reactor 330. The light receiver 122 is configured to receive the reflected light of the light emitted from the light source 121. The controller 25 obtains the glucose concentration CGlc based on the amount of received light by the light receiver 122 prior to the color change of the color-producing agent and the amount of light received by the light receiver after the color change of the color-producing agent.

In step S77, the controller 25 executes a process for feeding the collection liquid retained in the analyzing cartridge 30 to the liquid discharger 15. Specifically, the controller 25 executes a process for pumping air to the sodium detection reservoir 322 and the glucose detection reservoir 317 by reactuating the pump 142. The pumped air pushes the collection liquid through the discharge nipple 151 toward the flow path 152 (refer to FIG. 11F), and the emitted collection liquid is collected retained in the discharge liquid tank 153via the flow path 152.

In step S78, the controller 25 obtains the blood glucose AUC using the obtained sodium ion concentration CNa, and glucose concentration CGlc via equation (1) below, and stores the blood glucose AUC in the ROM.

$$AUC = (C_{Glc} \times E + F) \times t/(C_{Na} \times G + H) \cdots (1)$$

In the equation, t represents the tissue fluid collection time, and t=60 minutes in the present embodiment. E represents a constant.

The principle of the calculation of the blood glucose AUC is described later.

In step S79, the controller 25 displays, on the display unit 23, the blood glucose AUC obtained in step S78, and the process returns to step S8.

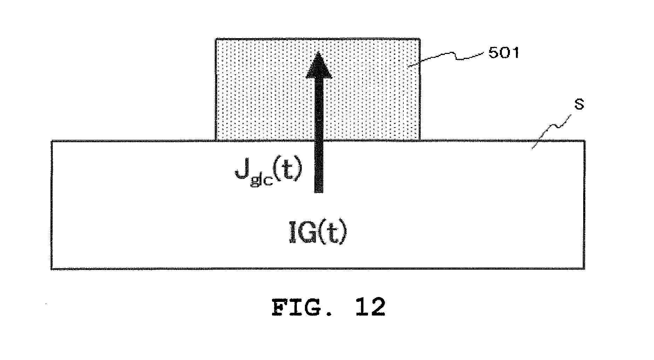

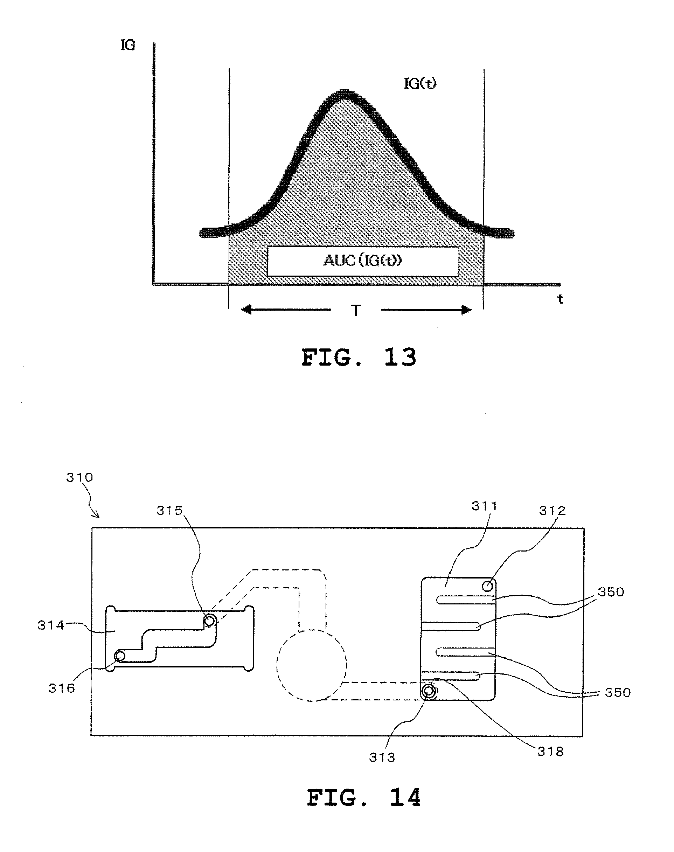

The principle of the blood glucose AUC measurement method is described below with reference to FIGS. 12 and 13.

Generally, the glucose concentration in tissue fluid (IG(t)) is known to closely track the glucose concentration in blood (BG(t)), and there is a known strong correlation between the glucose concentration in tissue fluid (IG(t)) and the glucose concentration in blood (BG(t)). The glucose concentration in tissue fluid (IG(t)) is can be represented by equation (2) below using the constant α.

$$BG(t) = \alpha \times IG(t) \cdots (2)$$

As shown in FIG. 12, when the gel 501 is adhered to the living body and tissue fluid is collected from the living body through the skin, the amount of glucose collected per unit time can be represented as glucose collection rate Jglc, and the glucose collection rate at a particular time t can be represented as Jglc(t). Jglc(t) at this time is represented as the sum of the glucose permeability Pglc and the tissue fluid glucose concentration IG(t) at time t, as shown in equation (3) below.

$$J_{glc}(t) = P_{glc} \times IG(t) \quad \cdots (3)$$

Note that the glucose permeability Pglc is a coefficient representing the permeability of the glucose relative to the skin, and the larger the glucose permeability Pglc, the greater the amount of glucose collected from the skin per unit time.

Consider now the case of a collection process performed for just a predetermined time T. On the left side of equation (3), when Jglc(t) is divided across the time T, the integral value becomes the total amount Mglt(t) of the glucose collected in the gel 501 from the living body within time T. The relationship can be expressed as shown in equation (4) below.

$$M_{glc}(T) = \int J_{glc}(t) \quad \cdots (4)$$

For example, when the glucose collection rate Jglc(t) = 10 ng/min, the total amount Mglc of the glucose collected in the gel 501 at time T=60 min becomes Mglt = 10 ng/min x 60 min = 600 ng.

On the right side of equation (3), when the glucose concentration in tissue fluid IG(t) is divided across time T, the value becomes the area of the figure (crosshatched area) stipulated by the graph of the glucose concentration IG(t) during time T (that is, the area under the curve AUC(IG(t))). The relationship can be expressed as shown in equation (5) below.

$$AUC(IG(t)) = \int IG(t) \quad \cdots (5)$$

As shown in equation (5), IG(t) and BG(t) have are correlated, and there is a. further correlation between the area under the curve AUC(IG(t)) and the area under the curve AUC(BG(t)). Accordingly, the relationship between the area under the curve AUC(IG(t)) and the area under the curve AUC(BG(t)) can be expressed by equation (6) below using the constant $\alpha$.

$$AUC(BG(t)) = \alpha \times AUC(IG(t)) \quad \cdots (6)$$

When considering the integral at time T, we can derive equation (7) below from equations (3) and (4).

$$M_{glc}(T) = \int P_{glc} \times IG(t) \quad \cdots (7)$$

Equation (8) below can be derived from equations (5) and (7).

$$M_{glc}(T) = P_{glc} \times AUC(IG(T)) \quad \cdots (8)$$

Mglc(T) can be expressed by equation (9) below using (BG(T)) via equations (6) and (8).

$$M_{glc} = (P_{glc} / \alpha) \times AUC(BG(T)) \quad \cdots (9)$$

That is, using equation (9) we can obtain AUC(BG(T)) from the constant $\alpha$, total amount Mglc(T) of the glucose accumulated in the gel 501 (refer to FIG. 12) within time T, and the permeability of the glucose relative to the skin at time T (glucose permeability Pglc). Note that ( may equals 1 since the blood glucose concentration and tissue fluid glucose concentration are virtually identical.

The principle for calculating glucose AUC based on equation (1) is described below.

The time area under the curve of blood glucose AUC(IG(T)) determined from the tissue fluid glucose can be determined from equation (10) below.

$$AUC(IG(T)) = Mglc(T) / P_{glc} \cdots (10)$$

Mglc(T) is the amount of glucose accumulated in the gel 501 within time T (amount of accumulated glucose). The amount of accumulated glucose is proportional to the glucose concentration Cglc in the collection liquid when the glucose has moved from the gel 501 to the collection liquid if the collection liquid volume is constant. Thus, Mglc(T) can be expressed by equation (11) below using the constants E and F.

$$M_{glc}(T) = C_{glc} \times E + F \cdots (11)$$

Pglc represents the ease of collecting the tissue fluid. The amount of collected tissue fluid changes depending on the condition of the skin (ease of collection). The amount of glucose contained in the tissue fluid changes depending on the glucose value. Therefore, the degree of collection of tissue fluid must be comprehended. The concentration of the sodium ions in the body is considered to be constant, unlike glucose. That is, a large amount of sodium ions contained in the collected tissue fluid is considered to indicate a good skin condition for the collection of tissue fluid. On the other hand, a low amount of collected sodium ions is considered to indicate a poor skin condition for the collection of tissue fluid.

The ease of collecting tissue fluid Pglc can be expressed by equation (12) below using the collection rate J of the sodium ions contained in the tissue fluid.

$$P_{glc} = J \times G + H \cdots (12)$$

The collection rate J is expressed by a value obtained by multiplying, by 1/t, the sodium ion concentration CNa of the sodium ions that moved from the gel 501 using the concentration of the sodium ions collected from the living body per unit time. Hence, we derive equation (13) below.

$$J = C_{Na} \times 1/t \cdots (13)$$

Equation (14) is derived from equations (10) through (13).

$$AUC(IG(T)) = (C_{glc} \times E + F) / (C_{Na} \times 1/t) \times G + H \cdots (14)$$

When the right side of equation (14) is reorganized,

$$AUC = (C_{Glc} \times E + F) \times t / (C_{Na} \times G + H) \cdots (1)$$

and we derive equation (1).

Note that the embodiment and reference examples disclosed herein are in all aspects merely examples, and are not intended to be in any way limiting. The scope of the present invention is defined by the scope of the claims and not be the description of the embodiment or the reference examples, and includes all modifications within the scope of the claims and the meanings and equivalences therein.

For example, although the present embodiment has been described by way of example in which a gel is used as the collecting body for collecting the tissue fluid, the present invention is not limited to this example inasmuch as tissue fluid may also be collected by material other than a gel if the material is adhered to the skin and possesses characteristics for holding the tissue fluid itself. For example, water absorbing paper, sponge, absorbent cotton, or a mesh sheet may also be used as the collecting body.

Although the present embodiment has been described by way of example in which pure water is used as the collection liquid, the present invention is not limited to this example inasmuch as a material other than pure water may also be used if the material is a liquid that is substantially without impurities that may affect detection of the analytes to be detected. When detecting glucose and sodium ion as in the present embodiment, a material may be used that does not affect detection by bonding to the sodium ions, for example a liquid that contains positive ions such as potassium ions. Note that, although the effect of osmotic pressure on the diffusion of the tissue fluid in the gel may be considered, insufficient diffusion efficiency may occur when the osmotic pressure is small due to the difference in concentration of components contained in the tissue fluid. The difference in concentration between the collection liquid and the gel may be increased to facilitate diffusion of the tissue fluid in the gel by adding beforehand a high concentration of material that does not affect the detection in the collection liquid.

Although the present embodiment has been described by way of example in which the apparatus body 10 and the analyzing cartridge 30 are separate bodies, the present invention is not limited to this example. For example, a structure equivalent to the analyzing cartridge 30 may be incorporated in the apparatus body 10.

Although the gel accommodating part 311 for accommodating the gel 501, and the detectors (glucose detector 21 and sodium ion detector 22) for detecting the analytes (sodium ions and glucose) are installed separately in the embodiment, the present invention is not limited to this example. For example, a configuration also may be used in which analytes are detected in the collection liquid retained in the gel accommodating part 311 after the analytes have moved to the collection liquid from the gel held in the gel accommodating part 311 without moving the collection liquid.

The configuration of the analyzing cartridge 30 of the present embodiment is not limited to the description referring to FIGS. 7 and 8, and may be variously modified. For example, a plurality of walls 350 may be provided in the gel accommodating part 311, as shown in FIG. 14. According to this configuration, the collection liquid may unevenly distribute in the gel accommodating part 311, and air present in the gal accommodating part 311 can be effectively pushed from the gel accommodating part to effectively prevent a mixture of air bubbles.

Although the above embodiment has been described by way of example in which the glucose detection reservoir and sodium ion detection reservoir are provided as separate reservoirs, the present invention is not limited to this example inasmuch as a single reservoir may be provided in the analyzing cartridge, so as to detect and analyze the glucose and sodium ions present in the liquid retained in this single reservoir.

Although the above embodiment has been described by way of example in which glucose detector 21 and the sodium detector 22 are stacked in a vertical direction, the present invention is not limited to this example inasmuch as, for example, the glucose detector 22 and the sodium detector 22 may be placed side by side in a horizontal deployment.

Although the above embodiment has been described by way of example in which the glucose detector 21 optically measures the glucose concentration, the present invention is not limited to this configuration inasmuch as, for example, a configuration may be used in which the glucose concentration is electrically measured. For example, the glucose detector 21 may be provided with an working electrode configured by a GOD enzyme film on a platinum electrode, and a counter electrode configured by a platinum electrode for electrically measuring glucose concentration. When measuring glucose concentration, these electrodes are immersed in the collection liquid retained in the glucose detection reservoir 317, and a current value is obtained by applying a constant voltage to the electrode. The glucose concentration can be obtained based on the obtained current value because the obtained current value increases dependent on the glucose concentration in the collection liquid oxidized by the GOD enzyme film formed on the platinum electrode.

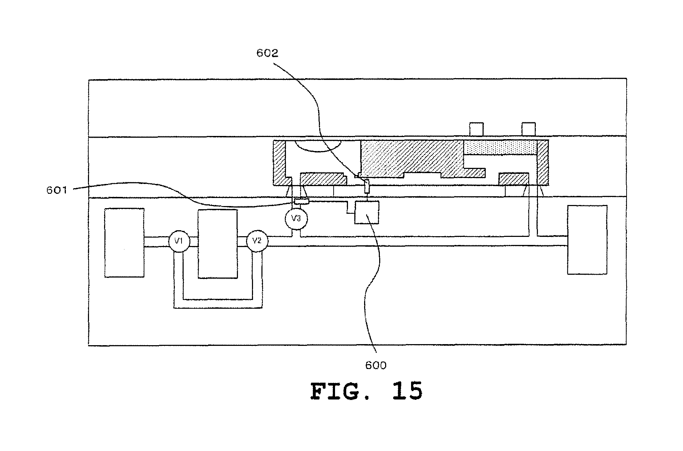

Although the above embodiment has been described by way of example in which the liquid feed to the analyzing cartridge 30 is controlled based on the volume of pumped air, the present invention is not limited to this configuration. For example, a sensor may be provided to detect whether the collection liquid has been fed to a predetermined position, so that the actuation of the pump can be stopped when the sensor has detected the liquid. Such an example is shown in FIG. 15. FIG. 15 shows a modification of the analyzing apparatus. This analyzing apparatus is provided with a positive terminal 601 protruding into the downstream path of the solenoid valve V3 of the downstream flow path 145, a negative terminal 602 protruding to the pedestal of the sodium detector 22, and a sensor 600 connected to both terminals. The sensor 600 is configured to apply a current to the positive terminal 601, and is capable of detecting a short circuit between the positive terminal 601 and the negative terminal 602.

As shown in FIG. 15, when the collection liquid reaches the first connection path 321 of the analyzing cartridge 30, the collection liquid short circuits the circuit between the positive terminal 601 and the negative terminal 602. The sensor 600 detects the short circuited terminals and outputs a detection signal to the controller 25 through a signal line not shown in the drawing, then the controller 25 receives the detection signal and stops the actuation of the pump 142.

According to this configuration, the sensor 600 reliably detects the liquid feed to the analyzing cartridge 30. In the above modification, the controller 25 may also output an error when a detection signal is not received from the sensor 600 within a predetermined time after the pump 142 has been actuated. When a detection signal has not been received within a predetermined time after the pump 142 has been actuated, an error may be output to alert the user that an analysis error has occurred because it is thought that the liquid transport 14 is faulty, poor contact between the injection nipple 141 and the induction port 142 or the like.

Although the above embodiment has been described by way of example in which a gel is used to collect the tissue fluid, the present invention is not limited to this example inasmuch as a material other than a gel may be used if the material is capable of maintaining a configuration that holds the tissue fluid obtained from a living body.

Although a gel configured by polyvinyl alcohol is used as the gel 501 in the above embodiment, a gel configured by cellulose or polyacrylic acid may also be used as the gel 501.

Although the above embodiment has been described by way of example in which the glucose AUC is calculated and output based on the measurement result of measuring the glucose concentration and sodium ion concentration, the present invention is not limited to this example inasmuch as just one of either the glucose concentration or sodium ion concentration may be measured and the measurement result output.

Although the above embodiment has been described by way of example in which glucose and sodium ions in tissue fluid are measured, the present invention is not limited to this example inasmuch as substances other than glucose and sodium ions contained in tissue fluid may be quantified. For example, biochemical components or drugs administered to the subject may be the substance measured by the present invention. One type of biochemical component are proteins such as albumin, globulin and the like, which may be considered as the chemical component. Biochemical components other than proteins include creatinine, creatine, uric acid, fructose, galactose, pentose, glycogen, lactic acid, pyruvic acid, ketone body and the like. Examples of drugs include digitalis, theophylline, arrhythmia agent, antiepileptics, amino glycoside antibiotic, glycopeptide antibiotic, antithrombotic, immunosuppressant and the like.

**Claims**

1. A method for analyzing an analyte in tissue fluid, comprising:

   placing a collecting member in contact with a skin of a subject, wherein the collecting member is configured to collect and retain tissue fluid containing an analyte from the subject;
   collecting tissue fluid from the subject into the collecting member;
   detaching the collecting member from the skin of the subject;
   causing the analyte in the tissue fluid collected by the collecting member to diffuse in a liquid by supplying the collecting member with the liquid; and
   detecting the analyte diffused in the liquid.

2. An analyzing apparatus for analyzing an analyte in tissue fluid, comprising:

   an accommodating part configured to accommodate a collecting member containing tissue fluid collected from a subject;
   a reservoir configured to retain liquid, wherein the reservoir is arranged in communication with the accommodating part;
   a liquid transporter configured to transport liquid to the accommodating part and to transport liquid to the reservoir from the accommodating part; and
   a detector for detecting an analyte in the liquid transported into the reservoir by the liquid transporter.

3. The analyzing apparatus according to claim 2, further comprising:

   a cartridge comprising the accommodating part and the reservoir; and
   a cartridge holder configured to detachably hold the cartridge.

4. The analyzing apparatus according to claim 3, wherein the cartridge comprises a reagent.

5. The analyzing apparatus according to claim 4, wherein
   the reagent causes a change in optical characteristics by reacting with the analyte in the liquid retained in the reservoir; and
   the detector is configured to detect the analyte based on the change in the optical characteristics of the reagent.

6. The analyzing apparatus according to any one of claims 2 to 5, wherein
   the detector comprises an electrode configured to contact the liquid retained in the reservoir.

7. The analyzing apparatus according to any one of claims 2 to 6, wherein
   the reservoir comprises a first reservoir and a second reservoir; and
   the detector comprises a first detector for detecting a first analyte in the liquid retained in the first reservoir, and a second detector for detecting a second analyte in the liquid retained in the second reservoir.

8. The analyzing apparatus according to claim 7, wherein
the accommodating part and the first reservoir are connected via a flow path;
the second reservoir is disposed in the flow path; and
the first reservoir comprises a reagent.

9. The analyzing apparatus according to claim 8, wherein
the first detector is disposed opposite a first surface of the cartridge set in the cartridge holder; and
the second detector is disposed opposite a second surface of the cartridge set in the cartridge holder.

10. The analyzing apparatus according to any one of claims 3 to 5, wherein
the cartridge has a channel that communicates with the accommodating part and the reservoir when the cartridge is set in the cartridge holder.

11. The analyzing apparatus according to any one of claims 2 to 10, wherein
the liquid transporter is configured to move the liquid in the accommodating part to the reservoir by supplying air to the accommodating part.

12. The analyzing apparatus according to any one of claims 7 to 9, further comprising
a controller for performing analysis relating to the first analytes based on the first analyte detection results obtained by the first detector and the second analyte detection results obtained by the second detector.

13. A cartridge configured to be detachably installed in an analyzing apparatus for analyzing an analyte in tissue fluid, the cartridge comprising:

an accommodating part configured to accommodate liquid supplied from the apparatus and a collecting member containing tissue fluid collected from a subject so that the collecting member is in contact with the liquid; and
a reservoir arranged in communication with the accommodating part, wherein the reservoir is configured to retain liquid transferred from the accommodating part.

14. An analyzing kit for analyzing an analyte in tissue fluid, comprising:

a collecting member for collecting and retaining tissue fluid collected from a subject; and
the cartridge according to claim 13.

15. The kit according to claim 14, wherein the collecting member comprises:

a collecting body for collecting and retaining tissue fluid collected from a subject; and
an adhesive film for holding the collecting body;
wherein
the collecting body is accommodated in the accommodating part by adhering the adhesive film to the margin of the opening when the collecting body is inserted through the opening of the accommodating part.

**FIG. 1**

FIG. 2

FIG. 3

FIG. 4

FIG. 5

50

502

501

**FIG. 6A**

50

502

503

501

**FIG. 6B**

FIG. 7

FIG. 8

START

Extraction preprocess — S1

Micropore formation — S2

Adhere collecting member — S3

120 minute
time course

Remove collecting member — S4

Adhere collecting member
to the cartridge — S5

Deploy cartridge
on the detector — S6

Analyze — S7

Confirm analysis result — S8

END

FIG. 9

Step S7

```
                    │
                    ▼
        ┌───────────────────────┐◄──────────┐
        │                       │  S71       │
      ◄─┤  Measurement start    ├─►          │
        │   instruction?        │  NO         │
        └───────────────────────┘─────────────┘
                    │
                   YES
                    ▼
        ┌───────────────────────┐  S72
        │ Feed collecting liquid to│
        │  gel accommodating part │
        └───────────────────────┘
                    │
                    ▼
        ┌───────────────────────┐◄──────────┐
        │       S73             │           │
        │     Predetermined      ├─►          │
        │     time elapsed?      │  NO         │
        └───────────────────────┘─────────────┘
                    │
                   YES
                    ▼
        ┌───────────────────────┐  S74
        │  Move collecting liquid │
        │     to reservoir       │
        └───────────────────────┘
                    │
                    ▼
        ┌───────────────────────┐  S75
        │    Measure sodium      │
        │    concentration       │
        └───────────────────────┘
                    │
                    ▼
        ┌───────────────────────┐  S76
        │    Measure glucose     │
        │    concentration       │
        └───────────────────────┘
                    │
                    ▼
        ┌───────────────────────┐  S77
        │    Discharge liquid    │
        └───────────────────────┘
                    │
                    ▼
        ┌───────────────────────┐  S78
        │   Calculate glucose AUC │
        └───────────────────────┘
                    │
                    ▼
        ┌───────────────────────┐  S79
        │   Display analysis     │
        │      results           │
        └───────────────────────┘
                    │
                    ▼
```

To Step S8

# FIG. 10

**FIG. 11A**

**FIG. 11B**

FIG. 11C

FIG. 11D

FIG. 11E

FIG. 11F

FIG. 11G

$J_{glc}(t)$

$IG(t)$

501

S

FIG. 12

FIG. 13

FIG. 14

FIG. 15

Europäisches
Patentamt

European
Patent Office

Office européen
des brevets

# EUROPEAN SEARCH REPORT

Application Number

EP 10 16 3395

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 2 198 774 A1 (SYSMEX CORP [JP]) 23 June 2010 (2010-06-23) * paragraph [0018] - paragraph [0019] * * paragraph [0026] * * paragraph [0075] - paragraph [0079] * * figures 6,32-34 * | 1,2,6 | INV. A61B5/00 |
| X | WO 2007/053186 A2 (LABNOW INC [US]; UNIV TEXAS [US]; MCDEVITT JOHN T [US]; CHRISTODOULIDE) 10 May 2007 (2007-05-10) * page 3, lines 20-28 * * page 45, line 23 - page 47, line 6 * * page 49, line 13 - page 50, line 2 * * page 51, line 27 - page 53, line 6 * * figures 2,8C,10A, * | 1-5,7-15 | |
| A | US 2003/224523 A1 (THORNBERG JOHN HERBERT [US] ET AL) 4 December 2003 (2003-12-04) * paragraphs [0023], [0024] * * paragraph [0029] * * paragraph [0034] - paragraph [0043] * * paragraphs [0059], [0060] * * paragraph [0063] * * paragraphs [0070] - [0075] * * figures 1-9 * | 1,2,13 | TECHNICAL FIELDS SEARCHED (IPC) A61B |
| A | US 2005/169799 A1 (SAWA KENNICHI [JP] ET AL) 4 August 2005 (2005-08-04) * paragraph [0050] - paragraph [0057] * * figures 6-8 * | 1,2,13 | |
| A | US 2007/027383 A1 (PEYSER THOMAS A [US] ET AL) 1 February 2007 (2007-02-01) * paragraph [0063] * * paragraph [0079] * * paragraph [0080] * * paragraph [0106] * * paragraph [0107] * * figures 2B,6,7 * | 1,2,13 | |

The present search report has been drawn up for all claims

| Place of search | Date of completion of the search | Examiner |
|---|---|---|
| Berlin | 21 September 2010 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 16 3395

This annex lists the patent family members relating to the patent documents cited in the above-mentioned European search report.
The members are as contained in the European Patent Office EDP file on
The European Patent Office is in no way liable for these particulars which are merely given for the purpose of information.

21-09-2010

| Patent document cited in search report | | Publication date | Patent family member(s) | | Publication date |
|---|---|---|---|---|---|
| EP 2198774 | A1 | 23-06-2010 | CN 101762629 A | | 30-06-2010 |
| | | | JP 2010169662 A | | 05-08-2010 |
| | | | US 2010160758 A1 | | 24-06-2010 |
| WO 2007053186 | A2 | 10-05-2007 | AU 2006309284 A1 | | 10-05-2007 |
| | | | CA 2610793 A1 | | 10-05-2007 |
| | | | EP 1910824 A2 | | 16-04-2008 |
| | | | US 2009215072 A1 | | 27-08-2009 |
| | | | ZA 200811082 A | | 30-06-2010 |
| US 2003224523 | A1 | 04-12-2003 | AU 2003240941 A1 | | 19-12-2003 |
| | | | CA 2487350 A1 | | 11-12-2003 |
| | | | EP 1508040 A1 | | 23-02-2005 |
| | | | JP 2005528610 T | | 22-09-2005 |
| | | | WO 03102577 A1 | | 11-12-2003 |
| US 2005169799 | A1 | 04-08-2005 | CN 1650795 A | | 10-08-2005 |
| | | | EP 1561418 A1 | | 10-08-2005 |
| | | | JP 2008183409 A | | 14-08-2008 |
| US 2007027383 | A1 | 01-02-2007 | US 2009270704 A1 | | 29-10-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9502357 A
- US 20050169799 A
- US 20070233011 A